(19) Europäisches Patentamt
European Patent Office
Office européen des brevets

(11) Veröffentlichungsnummer : **0 051 739**
**B1**

# (12) EUROPÄISCHE PATENTSCHRIFT

(45) Veröffentlichungstag der Patentschrift : **24.08.83**

(51) Int. Cl.³ : **C 07 C102/00**, C 07 C103/90

(21) Anmeldenummer : **81107899.7**

(22) Anmeldetag : **05.10.81**

## (54) Verfahren zur Herstellung von N,N,N',N'-Tetraacetylethylendiamin.

(30) Priorität : **08.11.80 DE 3042148**

(43) Veröffentlichungstag der Anmeldung : **19.05.82 Patentblatt 82/20**

(45) Bekanntmachung des Hinweises auf die Patenterteilung : **24.08.83 Patentblatt 83/34**

(84) Benannte Vertragsstaaten : **AT BE CH DE FR GB IT LI NL SE**

(56) Entgegenhaltungen :
**EP A 0 004 919**
**EP A 0 008 369**
**FR A 2 136 340**

(73) Patentinhaber : **BASF Aktiengesellschaft**
**Carl-Bosch-Strasse 38**
**D-6700 Ludwigshafen (DE)**

(72) Erfinder : **Koehler, Waldemar, Dr.**
**Max-Slevogt-Strasse 28**
**D-6710 Frankenthal (DE)**
Erfinder : **Wostbrock, Karl-Heinz, Dr.**
**Raiffeisenstrasse 9**
**D-6521 Moerstadt (DE)**
Erfinder : **Saladin, Guenter**
**Muenchbuschweg 73**
**D-6700 Ludwigshafen (DE)**
Erfinder : **Holzknecht, Bernhard, Dr.**
**Haardtstrasse 12**
**D-6701 Ellerstadt (DE)**

**EP 0 051 739 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

Verfahren zur Herstellung von N,N,N',N'-Tetraacetylethylendiamin

Gegenstand der Erfindung ist ein Verfahren zur Herstellung von N,N,N'N'-Tetraacetylethylendiamin (TAED) aus N,N'-Diacetylethylendiamin (DAED) und Essigsäureanhydrid :

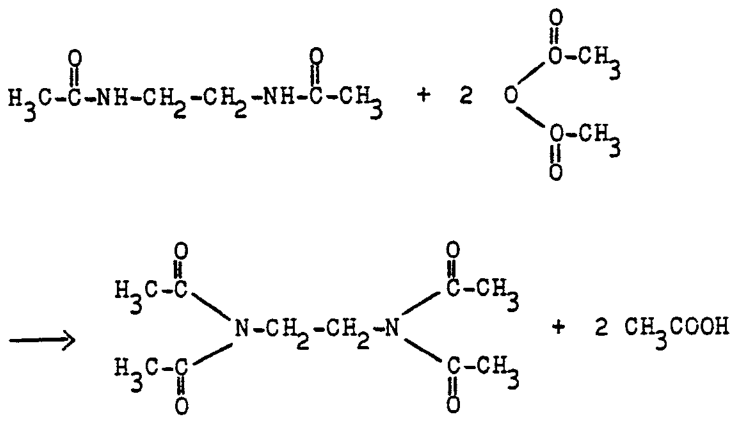

Tetraacetylethylendiamin wird als Kaltbleichaktivator für perborathaltige Waschmittel verwendet und kann auf verschiedene Weise erhalten werden :

Die DE-OS 19 10 300 beschreibt die Umsetzung von Ethylendiamin bzw. Diacetylethylendiamin mit Keten. Nach der DE-AS 20 52 822 werden Keten und Essigsäureanhydrid gleichzeitig als Acetylierungsmittel verwendet. Die Umsetzung von DAED mit Essigsäureanhydrid allein ist bereits aus Rec. Trav. Chim., *30* (1911), Seiten 183 bis 185, bekannt.

Bei diesen Verfahren entsteht immer ein Gemisch, das neben dem Zielprodukt noch unvollständig umgesetzte Bestandteile wie Diacetyl- und Triacetylethylendiamin sowie unerwünschte, insbesondere rückstandsbildende Nebenprodukte enthält.

Zur Isolierung des Zielproduktes aus dem Gemisch sind ebenfalls mehrere Wege beschrieben : Aus der DE-AS 21 18 281 ist bekannt, daß Tetraacetylethylendiamin kontinuierlich durch Umsetzen von N,N'-Diacetylethylendiamin mit im Überschuß angewandtem Essigsäureanhydrid bei 120 bis 170 °C hergestellt werden kann, indem man die gebildete Essigsäure fortwährend aus dem Reaktionsgemisch abdestilliert, dieses abkühlt, wobei TAED auskristallisiert, das Kristallisat abtrennt und einen Teil der Mutterlauge zurückführt. Die vollständige Rückführung der Mutterlauge verbietet sich, da sich sonst die Rückstände immer stärker anreichern und schließlich das Kristallisat verunreinigen würden. Dieses Verfahren hat somit den Nachteil, daß gelöstes TAED sowie teilumgesetzte Zwischenverbindungen entweder verloren gehen oder auf umständliche Weise zurückgewonnen werden müssen.

Zur Vermeidung dieser Nachteile ist in der DE-OS 28 16 174 vorgeschlagen worden, entweder das Reaktionsgemisch vor dem Auskristallisieren zu reinigen, oder die nach der Kristallisation anfallende Mutterlauge vor der Rückführung von Verunreinigungen zu befreien. Als geeignete Maßnahmen werden zum Beispiel genannt :

— eine Extraktion oder eine Adsorption an festen Adsorbentien,

— für die Reinigung der Mutterlösung nach der Kristallisation auch eine zweistufige Destillation unter vermindertem Druck, bei der zuerst überschüssiges Essigsäureanhydrid und Essigsäure und anschließend ein bei 50-90 °C erstarrendes Produktgemisch vom Rückstand abdestilliert wird, das zurückgeführt werden kann.

Auch der Vorschlag der DOS 28 16 174 befriedigt nicht ; abgesehen davon, daß nicht im einzelnen angegeben ist, wie die Extraktion auszuführen wäre, wäre sie mit einem erheblichen Aufwand zur Rückgewinnung des Extraktionsmittels verbunden. Die Entfärbung mit Hilfe von festen Adsorbentien bedeutete einen beachtlichen Verlust an Adsorbermaterial oder einen entsprechend hohen Aufwand für dessen Regenerierung. Die destillative Reinigung der Mutterlauge nach den Angaben der DE-OS 28 16 174 hat ebenfalls Nachteile, da die Kristallisation des Zielprodukts in Gegenwart aller Verunreinigungen erfolgt und somit seine Reinheit und Farbqualität beeinträchtigt wird. Erschwerend ist die Notwendigkeit einer zweistufigen Destillation, wobei der zweite Destillationsschritt bei besonders geringem Druck durchzuführen ist. Verfahren bei einem Druck im Bereich von 1 mbar sind im technischen Maßstab nur mit einem entsprechenden Aufwand durchführbar und daher unerwünscht.

Die Erfindung hat sich die Aufgabe gestellt, N,N,N',N'-Tetraacetylethylendiamin aus N,N'-Diacetylethylendiamin und im Überschuß angewandtem Essigsäureanhydrid unter Gewinnung von Essigsäure herzustellen, wobei mit geringem Destillationsaufwand reines kristalliertes Tetraacetylethylendiamin in hoher Ausbeute anfallen sollte.

Es wurde gefunden, daß diese Aufgabe dadurch gelöst werden kann, daß die im Reaktionsgemisch enthaltenen Bestandteile, die bis zu einer Temperatur von 190 °C bei 3 mbar flüchtig sind, aus dem

Reaktionsgemisch abgetrennt, kondensiert und zur Kristallisation gebracht werden. Diese Verfahrensweise kann absatzweise oder fortlaufend (kontinuierlich) gestaltet werden.

Der allgemeine Vorteil des erfindungsgemäßen Verfahrens besteht darin, daß das Zielprodukt in fester Form aus einem gereinigten Reaktionsgemisch gewonnen wird, und daß die Mutterlauge vollständig zurückgeführt werden kann, ohne daß zusätzliche Hilfsstoffe benötigt werden.

Der Ablauf der erfindungsgemäßen Verfahrens ergibt sich im einfachsten Fall aus Abbildung 1 für eine kontinuierliche Verfahrensführung :

Einem ein- oder mehrstufigen Reaktor (A) werden N,N'-Diacetylethylendiamin (1) sowie Essigsäureanhydrid (3) im Überschuß zugeführt. Als DAED verwendet man zweckmäßig eine technische Qualität, wobei geringe Mengen von Wasser und Essigsäure nicht stören. Man kann etwa das DAED enthaltende Reaktionsprodukt einer vorgeschalteten Stufe, in der Ethylendiamin mit Essigsäure umgesetzt werden, unmittelbar verwenden.

Die Menge an Essigsäureanhydrid (3) setzt sich zusammen aus dem zurückgewonnenen (6) und dem frischen Essigsäureanhydrid (2). Vorteilhaft erhält man im Reaktor im zeitlichen Gleichgewicht einen Überschuß vom 5- bis 15-fachen über die rechnerisch erforderliche stöchiometrische Menge aufrecht.

Die Umsetzung verläuft in bekannter Weise, bei etwa 120 bis 170 °C, wie dies in der DAS 21 18 281 beschrieben ist. Die gebildete Essigsäure wird fortlaufend aus dem Reaktionsgemisch abdestilliert, wobei Essigsäureanhydrid mitverdampft. Essigsäure und Essigsäureanhydrid (Strom 4) werden in der Destillationsstufe (D) fraktioniert. Die abgetrennte Essigsäure (Strom 5) kann z. B. für die Umsetzung mit Ethylendiamin zur Gewinnung von Diacetylethylendiamin verwendet werden.

In der Rückstandsabtrennung (B) wird das an Essigsäure bereits verarmte Reaktionsgemisch (7) in flüchtige (8) und nichtflüchtige Bestandteile (9) getrennt. Da sich beim Verdampfen der flüchtigen Bestandteile leicht zusätzliche Rückstände bilden, ist es zweckmäßig, Verdampfer mit kurzer Verweilzeit einzusetzen. In Frage kommen insbesondere Fallfilm- oder Dünnschichtverdampfer und sogenannte Flash-Verdampfer. Eine Möglichkeit zur schonenden Verdampfung der flüchtigen Komponenten bei gleichzeitiger Verfestigung und Granulierung des Rückstands ist in der DE-OS 24 52 805 für die Abtrennung von Phosgenierungsrückständen bei der Herstellung von Toluylendiisocyanat beschrieben. Dieses Verfahren läßt sich auch im vorliegenden Falle anwenden.

Das rückstandsfreie Reaktionsgemisch (8) kann nun in üblicher Weise (C) durch Kristallisation in Zielprodukt (10) und Mutterlauge (11) getrennt werden.

Eine besonders vorteilhafte Maßnahme zur schonenden Verdampfung empfindlicher Wertstoffe ist der — an sich bekannte — Zusatz eines sogenannten Strippmittels (Abstreifmittels), durch das bei vorgegebenem Gesamtdruck die Partialdrucke der Wertstoffe abgesenkt werden. Analog zur bekannten Wasserdampfdestillation wird erfindungsgemäß Essigsäure und/oder Essigsäureanhydrid als Strippmittel verwendet ; dies hat den Vorteil, daß keine Fremdstoffe zugegeben werden müssen und die Kondensation der verdampften Komponenten nicht erschwert wird. Dieses Verfahren wird durch Abbildung 2 wiedergegeben, die wie folgt erläutert wird :

Die im Reaktor (A) gebildete, anhydridhaltige Essigsäure wird dampfförmig (4) dem Verdampfer (B) zugeführt, der seinerseits auf einer Temperatur von z. B. 150 bis 190 °C gehalten wird. Die Brüden (8) werden kondensiert und TAED in (C) auskristallisiert. Die Mutterlaugen gehen in eine Destillation, wo Essigsäure abgetrennt und der Rest (13) dem Reaktor (A) wieder zugeführt wird.

Bei der Kondensation der Brüden kann in beiden Fällen je nach Kondensattemperatur die Löslichkeit des TAED im Kondensat überschritten werden. Um Belegung oder Verstopfung des Kondensators mit festem TAED zu vermeiden, empfiehlt sich die Verwendung eines sogenannten Quenchkühlers, bei dem die Brüden durch direkten Kontakt mit vorgekühltem Brüdenkondensat kondensiert und damit ein Auskondensieren der Brüden an kalten Wandflächen vermieden wird. Eine andere Möglichkeit, die vorzeitige Abscheidung von festem TAED zu vermeiden, ist eine zweistufige Kondensation : Der größte Teil der Brüden wird bei einer Temperatur oberhalb der Löslichkeitsgrenze von TAED im anfallenden Flüssigkeitsgemisch oder oberhalb der Schmelztemperatur von TAED kondensiert, der nur noch wenig TAED enthaltende Rest anschließend bei niedriger Temperatur.

Die Abscheidung von TAED in fester Form ist für sich nicht Gegenstand der Erfindung. Man kann sich der Technologie bedienen, wie sie aus der Lösungs- oder auch aus der Schmelzkristallisation bekannt ist (vgl. hierzu : Ullmanns Encyklopädie der technischen Chemie, 4. Auflage, Band 2, Seiten 676 bis 682). Falls das Brüdenkondensat (8) bereits festes TAED enthält, kann es zweckmäßig sein, dieses z. B. durch Aufwärmen wieder in Lösung zu bringen, um für die Abscheidung einheitliche Ausgangsbedingungen zu schaffen. Eine besonders vorteilhaft anzuwendende Methode zur Abscheidung des TAED in fester Form ist die in der GB-PS 1 083 850 und DE-OS 26 06 364 beschriebene Schichtkristallisation.

Das fest abgeschiedene TAED liegt je nach Wahl der Kristallisations- bzw. Erstarrungsmethode als feinkristallines, als körniges oder als massiv erstarrtes Material vor. Nach Abtrennung von der Mutterlauge z. B. durch Dekantieren, Filtrieren oder Abschleudern kann es notwendig sein, zur Entfernung noch anhaftender Mutterlauge nachzuwaschen. Zweckmäßigerweise verwendet man hierzu reine Essigsäure und/oder Essigsäureanhydrid ; beides kann anschließend den Mutterlaugen zugegeben werden.

Das noch feuchte feste Zielprodukt kann auf übliche Weise getrocknet werden. Es ist ausreichend hydrolysebeständig, so daß es mit Wasser bei bis zu 50 °C gewaschen und anschließend schonend

getrocknet werden kann. Die Mutterlaugen enthalten als gelöste Bestandteile restliches TAED sowie noch teilweise umgesetzte Produkte (Tri- und Diacetylethylendiamin), die wie eingehend erörtert, zurückgeführt werden.

Nach der Erfindung kann Tetraacetylethylendiamin mit einer Ausbeute von mehr als 95 %, bezogen auf Diacetylethylendiamin, gewonnen werden. Es fällt in rein weißer Form an, mit einer Reinheit von 99,0 % und mehr; der Rest ist hauptsächlich Triacetylethylendiamin.

Beispiel 1 (Verfahrensvariante gemäß Figur 1)

In einer Kaskade aus zwei geschlossenen Rührgefäßen mit je 1,5 l Füllinhalt werden fortlaufend 148 g/h Diacetylethylendiamin-Schmelze (Reinheit: 97,3 Gew.%), 212 g/h frisches Essigsäureanhydrid sowie zwei weitere, noch zu beschreibende Rückführströme eingeleitet. Die Temperatur wird in jedem der beiden Gefäße auf 140 °C gehalten. Aus beiden Gefäßen dampft ein Gemisch aus Essigsäure und Essigsäureanhydrid ab, das gemeinsam in den Mittelteil einer ebenfalls kontinuierlich betriebenen Füllkörper-Destillationskolonne geführt wird. Die am Kolonnenkopf als Destillat anfallende Essigsäure wird abgezogen, das in der Destillationsblase anfallende Essigsäureanhydrid läuft in das zweite Rührgefäß der Kaskade zurück.

Das aus dem zweiten Gefäß der Kaskade abfließende Reaktionsgemisch wird in einem Vorratsbehälter zwischengelagert und von dort aus fortlaufend und gleichmäßig in einen bei einem Druck von 7 mbar und mit einer Badtemperatur von 225 °C betriebenen Rotationsverdampfer geführt. Im Rotationsverdampfer verbleibt ein brauner, zähflüssiger, teilweise fester Verdampfungsrückstand. Die aus dem Rotationsverdampfer abziehenden Brüden werden über eine auf 180 °C gehaltene Leitung und einen absteigenden, auf 150 °C gehaltenen Kühler in einen auf 30 °C gekühlten, als Kristallisationsgefäß dienenden 2-Liter-Rührkolben eingeleitet. Zur Vermeidung von Verdampfungsverlusten an Essigsäureanhydrid ist dem Kristallisationsgefäß ein auf 10 °C thermostatierter Rückflußkühler aufgesetzt. Über diesen Kühler sind Kristallisationsgefäß und vorgeschalteter Rotationsverdampfer an eine Vakuumpumpe mit Manostaten angeschlossen, um den im Verdampfer gewünschten Unterdruck einzustellen.

Alle zwei Stunden wird der Inhalt des Kristallisationskolbens in eine Glasfilternutsche entleert, die Mutterlösung von Kristallisat abgesaugt, das Kristallisat mit 200 g Essigsäureanhydrid nachgewaschen und anschließend bei 80 °C im Vakuum-Trockenschrank getrocknet. Der aus der Gewichtsabnahme beim Trocknen ermittelte Verlust an Essigsäureanhydrid beträgt im Mittel 11 g/h und wird durch Zugabe der gleichen Menge zur Mutterlösung ergänzt. Die mit der Waschlösung vereinigte Mutterlösung wird in einem Zwischengefäß gesammelt und fortlaufend und gleichmäßig in das erste Reaktionsgefäß der Kaskade zurückgeführt.

Nach 6 bis 8 Rückführzyklen ist die Apparatur bezüglich der Mengenströme und ihrer Zusammensetzung in einem stationaren Zustand. Durch Bilanzierung im stationaren Zustand werden die folgenden Daten ermittelt: Es fallen im Mittel 221 g/h trockenes TAED-Kristallisat an, das eine Reinheit von 99,2 Gew.% mit 0,8 Gew.% Triacetylethylendiamin als Nebenbestandteil und eine APHA-Farbzahl von maximal 15 aufweist. Bezogen auf das eingesetzte DAED und unter Berücksichtigung der Reinheiten von Ausgangs- und Endprodukt beträgt die Ausbeute an TAED im Mittel 96,2 %. Im Kolben des Rotationsverdampfers sammeln sich etwa 8 g/h an Verdampfungsrückstand an. Die am Kopf der Destillationskolonne anfallende Menge an Essigsäure beträgt 125 g/h, die aus der Destillationsblase in das zweite Reaktionsgefäß rückgeführte Menge an Essigsäureanhydrid beträgt im Mittel 778 g/h. Die beim Abfiltrieren und Waschen des TAED-Kristallisats anfallende Mutter- und Waschlösung ist bei störungsfreiem Betrieb des Rotationsverdampfers farblos; es fallen hiervon im Mittel 670 g/h an. Aus den unter stationalen Bedingungen durch Bilanzierung ermittelten Mengenströmen ergibt sich für die Reaktionskaskade ein Molverhältnis von zugeführtem Essigsäureanhydrid zu zugeführtem Diacetylethylendiamin von etwa 15, das Molverhältnis an frisch zugeführtem Essigsäureanhydrid zu zugeführtem Diacetylethylendiamin beträgt 2,08.

Beispiel 2 (Verfahrensvariante gemäß Figur 2)

Wie im Beispiel 1 werden in die Rührkolben-Kaskade der gleichen Versuchsapparatur 148 g/h Diacetylethylendiamin-Schmelze (Reinheit: 97,3 Gew.%) und 212 g/h frisches Essigsäureanhydrid eindosiert; die Reaktionstemperatur beträgt ebenfalls 140 °C. Im Unterschied zur im Beispiel 1 demonstrierten Verfahrensführung, wird das aus dem Reaktionskolben abdampfende Gemisch aus Essigsäure und Essigsäureanhydrid nicht in die Destillationskolonne geleitet, sondern mittels eines absteigenden Kühlers zwischenkondensiert, in einem Auffangbehälter zwischengelagert und anschließend gemeinsam mit dem Reaktionsaustrag in den Rotationsverdampfer eindosiert. Die Zwischenkondensation des dampfförmigen Essigsäure/Essigsäureanhydrid-Gemisches hat lediglich laborapparative Gründe und erleichtert die Überführung dieses Mengenstromes in den Verdampfer. Die Heizbadtemperatur des Rotationsverdampfers beträgt 190 °C, der Verdampfungsdruck kann dank der Zugabe des Essigsäure/Essigsäureanhydrid-Gemisches als Strippmittel auf 20 mbar angehoben werden. Zur Kondensation der aus dem Rotationsverdampfer abziehenden Brüden wird der zum Kristallisationskolben führende, absteigende Kühler mit Wärmeträgeröl auf 100 °C gehalten. Die Kristallisationstemperatur beträgt 30 °C.

Die nachfolgenden Arbeitsgänge zur Abtrennung, Wäsche und Trocknung des TAED-Kristallisats sind die gleichen wie in Beispiel 1. Anschließend wird jedoch die vereinigte Mutter- und Waschlösung zur Abtrennung der darin enthaltenen Essigsäure in die Destillationskolonne geführt und die in der Kolonnenblase anfallende, essigsäurefreie Lösung in den ersten Reaktionskolben der Kaskade zurückgeleitet.

Die Bilanzierung unter stationaren Bedingungen ergab folgendes : Es fallen im Mittel 222 g/h trockenes TAED-Kristallisat mit einer Reinheit von 99,5 Gew.% und 0,5 Gew.% Triacetylethylendiamin als Nebenbestandteil an ; dies entspricht einer Ausbeute von 97,0 % an reinem TAED, bezogen auf das reine DAED im Ausgangsprodukt. Die APHA-Frabzahl des Produkts lag im Mittel bei 10. Im Rotationsverdampfer sammelten sich 6 bis 7 g/h an festem Rückstand an. Die am Kopf der Destillationskolonne anfallende Menge an Essigsäure betrug 125 g/h, der Sumpfaustrag der Kolonne lag bei 1 450 g/h. Es errechnet sich ein molares Verbrauchsverhältnis von frischem Essigsäureanhydrid zu Diacetylethylendiamin von 2.08 und ein Gesamteinsatzverhältnis von ca. 15 Molen frischem und rückgeführtem Essigsäureanhydrid je Mol Diacetylethylendiamin.

**Ansprüche**

1. Verfahren zur Herstellung von N,N,N',N'-Tetraacetylethylendiamin (TAED) durch Umsetzung von N,N'-Diacetylethylendiamin (DAED) mit überschüssigem Essigsäureanhydrid, unter Freisetzung von Essigsäure, Entfernung der gebildeten Essigsäure aus dem Reaktionsgemisch durch Destillation und Gewinnung des TAED durch Kristallisation, dadurch gekennzeichnet, daß man

a) aus dem Reaktionsgemisch das Essigsäureanhydrid zusammen mit Tetraacetylethylendiamin und den bis zu einer Sumpftemperatur von 190 °C bei 3 mbar flüchtigen Bestandteilen vom nichtflüchtigen Rückstand abdestilliert, kondensiert und

b) aus dem Kondensat durch Kristallisation Tetraacetylethylendiamin gewinnt sowie

c) die erhaltene Mutterlauge gegebenenfalls nach Abdestillieren der Essigsäure zurückführt.

2. Verfahren nach Anspruch 1, dadurch gekennzeichnet, daß die flüchtigen Bestandteile mit Hilfe dampfförmiger, bei der Reaktion anfallender Essigsäure und/oder Essigsäureanhydrid abgestreift werden.

**Claims**

1. A process for the production of N,N,N',N'-tetraacetyl ethylenediamine (TAED) by reacting N,N'-diacetyl ethylenediamine (DAED) with excess acetic anhydride, with liberation of acetic acid, removing the acetic acid formed from the reaction mixture by distillation, and recovering the TAED by crystallization, wherein

a) the acetic anhydride together with tetraacetyl ethylenediamine and the constituents which are volatile at a bottoms temperature of 190 °C at 3 millibars is distilled off from the reaction mixture, leaving non-volatile residue, and condensed, and

b) tetraacetyl ethylenediamine is recovered from the condensate by crystallization, and

c) the resulting mother liquor is returned to the reaction, if necessary after distilling off the acetic acid.

2. A process as claimed in claim 1, wherein the volatile constituents are stripped off with the aid of vaporous acetic acid and/or acetic anhydride formed in the reaction.

**Revendications**

1. Procédé de préparation de la N,N,N',N'-tétra-acétyl-éthylène-diamine (TAED) par réaction de la N,N'-diacétyl-éthylène-diamine (DAED) et d'anhydride acétique en excès avec formation d'acide acétique libre, élimination de l'acide acétique formé du mélange réactionnel par distillation et isolement de la TAED par cristallisation, caractérisé en ce que :

a) on sépare le mélange réactionnel, par distillation, en anhydride acétique, tétra-acétyl-éthylène-diamine et ingrédients volatils jusqu'à une température du résidu de distillation de 190 °C sous 3 millibars, d'une part, et en résidu non volatil d'autre part, et on condense la fraction volatile ;

b) on sépare la tétra-acétyl-éthylène-diamine du condensat par cristallisation et

c) on recycle la lessive mère résiduelle, éventuellement après élimination de l'acide acétique par distillation.

2. Procédé suivant la revendication 1, caractérisé en ce que les ingrédients volatils sont séparés par entraînement par l'acide acétique et (ou) l'anhydride acétique, formés durant la réaction, à l'état gazeux.

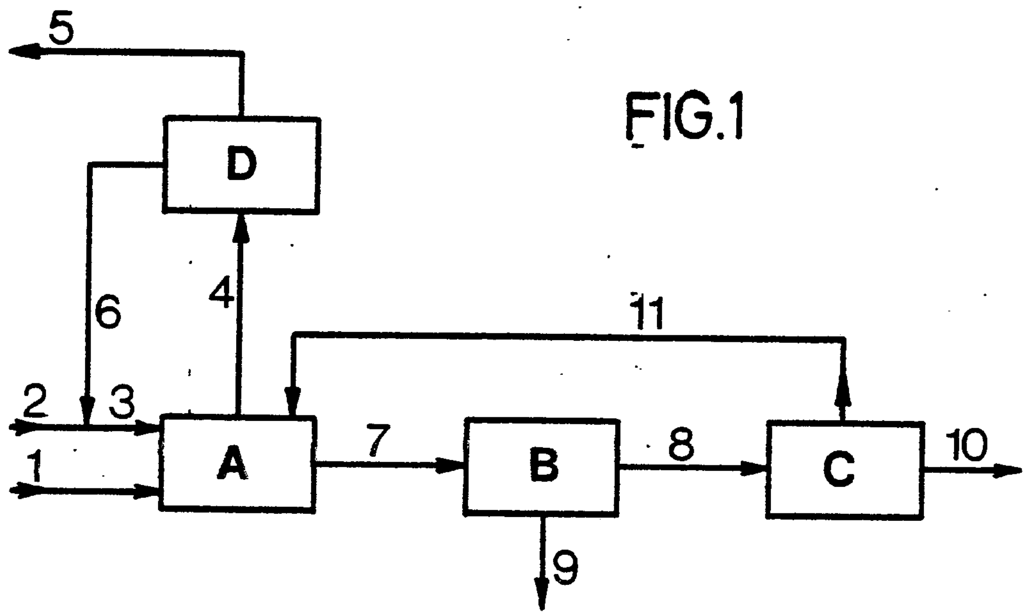


FIG.1

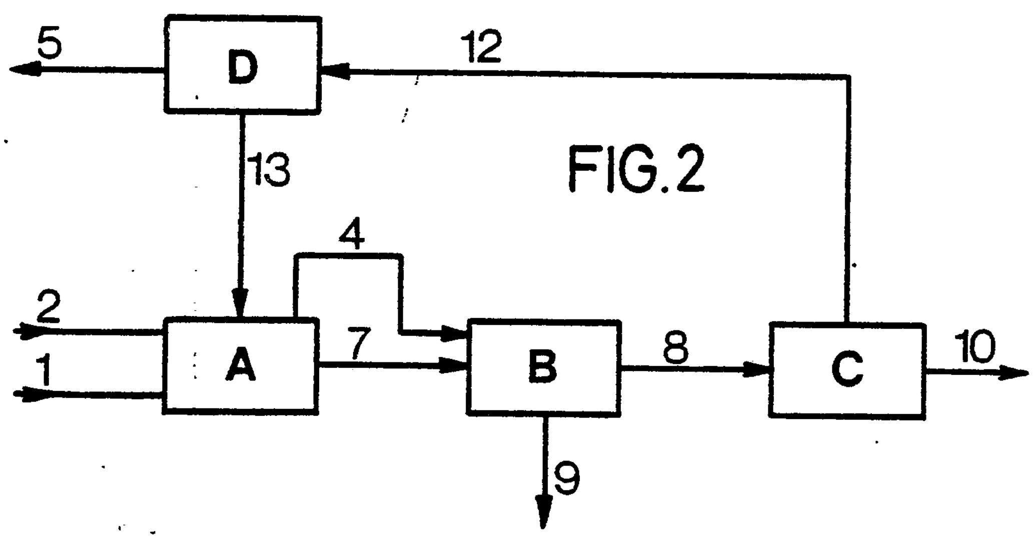


FIG.2